# EUROPÄISCHE PATENTSCHRIFT

(11) **EP 1 749 548 B1**
(45) Veröffentlichungstag und Bekanntmachung des Hinweises auf die Patenterteilung: **22.09.2010**
(21) Anmeldenummer: 06023415.0
(22) Anmeldetag: 30.08.2002
(51) Int. Cl.: A61M 11/00, G06F 19/00, G01F 1/34, G01F 1/58, A61M 11/06

(54) **Spender für Medien**
Dispenser for media
Distributeur pour milieux

(30) Priorität: 18.09.2001 DE 10146815
(43) Veröffentlichungstag der Anmeldung: 07.02.2007
(62) Teilanmeldung aus: 02019405.6
(73) Patentinhaber: Ing. Erich Pfeiffer GmbH, 78315 Radolfzell (DE)
(72) Erfinder: Fuchs, Karl-Heinz, 78315 Radolfzell (DE)
(74) Vertreter: Patentanwälte Ruff, Wilhelm, Beier, Dauster & Partner

(56) Entgegenhaltungen:
- EP-A- 0 923 957
- EP-A- 1 184 083
- DE-A- 19 835 941
- US-A- 6 138 669
- US-B1- 6 196 218

## Beschreibung

### Anwendungsgebiet und Stand der Technik

Die vorliegende Erfindung betrifft einen Spender für Medien, wie er insbesondere für den Austrag von Fluiden geeignet ist. Derartige Spender werden insbesondere für den Austrag von Fluiden, insbesondere durch zerstäubtes Versprühen, in wenigstens einer definierten Dosis verwendet, die wenigstens einen pharmazeutischen Wirkstoff enthalten. Das zerstäubte Austragen von derartigen Fluiden erfolgt dabei insbesondere intranasal, wobei vorzugsweise in jedes der beiden Nasenlöcher eine genau dosierte und gleichgroße Menge Medium ausgetragen wird. Bei den pharmazeutischen Wirkstoffen oder Wirkstoffkombinationen, die in dieser Art und Weise ausgetragen werden können, kann es sich um Opiate oder andere Wirkstoffe handeln, insbesondere Kopfschmerzmittel und Schmerzmittel sowie Medikamente für die Behandlung von chronischen Erkrankungen, die einer kontinuierlichen Anwendung (z. B. Diabetes, Herz- und Kreislauferkrankungen usw.) bedürfen. Die Eignung eines Wirkstoffes Wirkstoffes für einen derartigen Austrag hängt davon ab, dass der Wirkstoff über die Schleimhäute, insbesondere die Nasenschleimhäute, absorbiert werden kann. Dadurch ist eine rasche Absorption und ein rasches Verbringen in den Körper, insbesondere in den Zerebralbereich des Körpers, gewährleistet.

Derartige Spender weisen einen Vorratsbehälter für das entsprechende Medium auf. Der Vorratsbehälter ist über einen Medienpfad mit einer Austragöffnung, z. B. einer Sprühdüse, verbunden. Im Medienpfad ist eine betätigbare Fördereinrichtung vorgesehen, die das Medium vom Vorratsbehälter zur Austragsöffnung fördert. Ein Fördervorgang erfolgt dann, wenn ein Betätigungsmittel betätigt wurde.

Die bei einer Betätigung ausgetragene Menge Medium ist der Fördermenge der Fördereinrichtung bei einer Betätigung des Betätigungsmittels abhängig. In der Regel werden als Fördereinrichtung Kolbenpumpen verwendet, so dass die ausgetragene Menge Medium im wesentlichen dem Volumen der Arbeitskammer der Kolbenpumpe entspricht.

Dieses Maß ist aber nicht immer ein sicheres Maß dafür, wie groß die tatsächlich ausgetragene Menge ist. Zum einen können im Bereich des Pumpenkolbens noch Restluftmengen vorhanden sein. Dies ist nach längeren Lagerzeiten und vor der ersten Benutzung des Spenders der Fall. Dann ist ein sogenanntes "Priming" des Spenders erforderlich, bei dem durch mehrere Austragshübe nacheinander die Pumpe etc. vollständig mit Medium gefüllt wird. Zum anderen kann es vorkommen, dass bei einer Betätigung des Spenders kein vollständiger Betätigungshub durchgeführt wird. Zwar kann man durch konstruktive Maßnahmen erreichen, dass der Benutzer bei der Betätigung eines Spenders möglichst einen vollständigen Betätigungshub durchführt. Dennoch sind Fehlbetätigungen nicht auszuschließen.

Aus der EP 0 923 957 A1 ist ein Tröpfchenerzeuger bekannt geworden, der mittels einer von einem Piezokristall erzeugten Vibration ein Medium in Tröpfchen zerstäubt. Eine solche Einrichtung ist auch in der EP 1 184 083 A1 beschrieben, die auch zeigt, dass die von der Einrichtung erzeugten Tröpfchen dann von einem Luftstrom mitgenommen werden. Derartige Vorrichtungen sind hauptsächlich für Inhalatoren vorgesehen. Bei diesen Einrichtungen ist ein Differenzdrucksensor erwähnt, der eine Überwachung der Tröpfchenerzeugung ermöglicht und den Durchfluss sowie Funktionsstörungen durch eine leere Vibrationskammer oder Verstopfung detektieren kann.

### Aufgabe und Lösung

Aufgabe der Erfindung ist es, vorbekannte Spender dahingehend weiter zu entwickeln, dass der tatsächliche Volumenaustrag des Mediums bestimmbar ist.

Diese Aufgabe wird gelöst durch einen Spender nach dem Anspruch 1.

Mittels der vorgesehenen Erfassungseinrichtung kann für jede einzelne Betätigung des Spenders der tatsächlich Mengenausgetrag des Mediums erfasst werden. Die Erfassungseinrichtung enthält einen im Medienpfad angeordneten Durchflussmengenmesser, der im Medienpfad zwischen der Fördereinrichtung und der Austragsöffnung angeordnet ist, vorteilhaft an einer Stelle, an der stromaufwärts nur noch die Austrittsöffnung ein Strömungshindernis bildet. Dadurch ist sichergestellt, dass der Volumenstrom des Mediums erfasst wird, der auch tatsächlich durch die Austragöffnung strömt.

Bevorzugt sollte der Durchflussmengenmesser eine quantitative Durchflussmessung durchführen. Es kann sich um einen thermischen oder induktiven Durchflussmengenmesser oder um einen nach dem Druckdifferenzmessverfahren arbeitenden Durchflussmengenmesser handeln.

Der Messbereich des Messverfahrens sollte an das Volumen des Mediums angepasst sein, das mit einem Pumphub des Spenders ausgetragen werden soll. Dieses Volumen kann auch unterhalb von 200µl liegen, insbesondere im Bereich zwischen 30 und 150µl. Dabei soll auch ein Austrag erfasst werden, der innerhalb eines kurzen Zeitintervalls, z. B. im Bereich von 50 bis 100 Millisekunden erfolgt. Es sollte eine große Messgenauigkeit gewährleistet sein, da es sich bei derart ausgetragenen Medien oft um Medien handelt, die pharmazeutische Wirkstoffe enthalten, die sehr genau dosiert sein sollten, da sie in Überdosierung gesundheitsschädlich sein könnten.

Die Fördereinrichtung ist eine Kolbenpumpe, die z. B. als einfachwirkende Schubkolbenpumpe, ausgebildet sein kann. Mittels des Sensors kann eine wenigstens mittelbar den Hubweg des Hubkolbens repräsentierende Größe erfasst werden. So kann das Erreichen der oberen Totpunktlage mittels eines Endlagenschalters erfasst werden, beispielsweise eines Tastschalters, der vorzugsweise als Öffner ausgebildet ist. Alternativ oder ergänzend kann ein weiterer Endlagenschalter vorgesehen sein, der das Verlassen der betätigungsfrei eingenommenen unteren Totpunktlage der Kolbenpumpe erfasst.

Mittels des Endlagensensors, der das Erreichen der oberen Totpunktlage erfasst, kann festgestellt werden, ob eine vollständige Betätigung des Betätigungsmittels und somit ein vollständiger Betätigungshub der Kolbenpumpe stattgefunden hat. Diese Maßnahme kann dazu dienen, einen Durchflussmengenmesser abzugleichen und zu eichen. Wird ein vollständiger Kolbenhub durchgeführt, so müsste die vom Durchflussmengenmesser erfasste Durchflussmenge dem Volumen eines Kolbenhubes entsprechen. Hierbei kann beispielsweise dann, wenn zusätzlich noch das Verlassen der unteren Totpunktlage erfasst wird, festgestellt werden, ob der Austrag auch in einem einzigen, vollständig durchgehenden Betätigungshub durchgeführt wurde. Insbesondere diese Fälle können zum Abgleichen für das erfasste Signal des Durchflussmengenmessers dienen. Hierbei können insbesondere zeitliche Drifts und ähnliche Eigenschaften sowie auch die stochastische Streuung des Messsignals im Bereich des Messsensors teilweise kompensiert werden. Darüber hinaus wäre es auch möglich, mittels eines Wegsensors den zurückgelegten Weg des Pumpenkolbens zu erfassen. Aufgrund des diesem Hubweg entsprechenden verdrängten Volumens kann dann die Menge des ausgetragenen Mediums ermittelt werden. Dies gilt insbesondere bei flüssigen Medien, die inkompressibel sind oder als inkompressibel betrachtet werden können. Die durch die Wegmessung indirekt erfolgte Austragsmengenmessung kann ergänzend oder alternativ zu der Durchflussmengenmessung mittels eines Durchflussmengenmessers vorgenommen werden. Werden beide Meßmethoden angewandt, so kann auch hier jeweils ein Abgleich der beiden Messergebnisse erfolgen, wodurch es möglich sein kann, die Qualität des Messergebnisses und die Messgenauigkeit zu erhöhen.

Neben der Erfassung der ausgetragenen Medienmenge ist es auch möglich, in der Erfassungseinrichtung zugleich den Zeitpunkt der Betätigung des Spenders, die Zeit seit der letzten Betätigung des Spenders und/oder die Uhrzeit der letzten Betätigung bzw. das Datum und die Uhrzeit der letzten Betätigung zu erfassen. Diese Erfassung kann zum einen nur der Protokollierung der erfolgten Betätigungen dienen, andererseits kann auch vorgesehen sein, dass beispielsweise das austragbare Volumen in Abhängigkeit vorhergehender Betätigungen erfolgt. Hierbei kann beispielsweise vorgesehen sein, dass über eine Betätigung jeweils nur soviel Medium austragbar ist, dass die ausgetragene Menge über einen gewissen Zeitraum hinweg einen bestimmten vorgegebenen Wert nicht überschreitet. Dies kann beispielsweise dann angebracht sein, wenn es sich um ein Medium handelt, das süchtig-machende oder in Überdosis letal wirkende pharmazeutische Wirkstoffe beinhaltet und bei dem eine gewisse Dosierung über einen gewissen Zeitraum hinweg nicht überschritten werden soll. Soweit zumindest eine Protokollierung des Austragverhaltens des Spenders vorgenommen wird, sind diese Informationen vorzugsweise im Spender selbst, insbesondere im Bereich der Erfassungseinrichtung, so abgespeichert, dass sie abrufbar und/oder auslesbar abgelegt sind. Soweit das Austragvolumen einer nachfolgenden Betätigung des Spenders aufgrund vorhergehender Betätigungen des Spenders und eventuell aufgrund des seither abgelaufenen Zeitraumes erfolgt, ist vorzugsweise im Bereich des Spenders eine Verstelleinrichtung angeordnet, die den maximalen Hubweg der Kolbenpumpe begrenzt.

Es ist es auch möglich, im Bereich der Austragöffnung einen Messfühler, z. B. einen Temperatursensor, anzuordnen. Der Messfühler kann insbesondere in unmittelbarer Nähe der Spitze einer Nasenolive und damit der Austragöffnung des Spenders angeordnet sein. Er erfasst, ob eine Betätigung des Spenders erfolgt oder erfolgen soll, bei der der Spender an einem vorgesehenen Applikationsort, beispielsweise im Bereich eines Nasenflügels, angesetzt ist. Dadurch können unbeabsichtigte oder nicht vorgesehene Betätigungen und Nutzungsweisen des Spenders verhindert oder bei Mengenerfassungen nicht mit einzubezogen werden. Der Messfühler kann so ausgebildet sein, dass er eine Umgebungsgröße, wie die Temperatur, Luftfeuchtigkeit, Helligkeit oder dergleichen erfasst und keine große zeitliche Verzögerung bei den Messungen entsteht. Ein Temperatursensor sollte also beispielsweise nicht im Bereich einer großen thermischen Kapazität angeordnet sein, um eine Veränderung der Umgebungstemperatur rasch erfassen zu können.

In Abhängigkeit der Messsignale des Messfühlers ist es somit möglich, darauf zu schließen, ob die Applikation des Mediums am vorgesehenen Applikationsort, beispielsweise der Nase, erfolgt. Es ist darüber hinaus möglich, eine Betätigungssperre dann zu aktivieren, wenn festgestellt wird, dass eine Applikation nicht am vorgesehenen Applikationsort erfolgt, bzw. dass eine Applikation nur dann ermöglicht wird, wenn vorausgesetzt werden kann, dass eine Applikation am beabsichtigten Applikationsort durchgeführt wird. Andererseits oder ergänzend ist es auch möglich, in der Erfassungseinrichtung bei der Bemessung möglicher nachfolgender Betätigungen des Spenders das Volumens so zu bemessen, dass nur wahrscheinlich am vorgesehenen Applikationsort durchgeführte Austräge berücksichtigt werden.

Als Messfühler kann außer einen Temperatursensor auch optische Sensortechnik oder eine mit Messung der Zusammensetzung der Umgebungsluft (Luftfeuchtigkeit, CO₂-Gehalt) arbeitende verwendet werden.

Mittels des Messfühlers kann nicht nur erfasst werden, an welcher Stelle eine Applikation des Mediums erfolgt, es ist auch der zeitliche Verlauf der Temperatur des Mediums bzw. des Lagerbereichs des Mediums erfassbar. Insbesondere bei thermisch sensiblen Medikamenten kann so sichergestellt werden, dass nicht weiter Medien ausgetragen werden, die sich beispielsweise aufgrund thermischer Einflüsse zwischenzeitlich verändert haben und nicht mehr zur Verwendung geeignet sind.

Ergänzend oder alternativ zu einer Verstelleinrichtung für das Austragvolumen ist es auch möglich, dass der Spender eine schaltbare Betätigungssperre aufweist. Diese Betätigungssperre kann insbesondere ein nochmaliges Betätigen des Spenders innerhalb eines Zeitintervalls verhindern. Dabei kann das Zeitintervall in Abhängigkeit von in der Erfassungseinrichtung erfassbaren Größen, beispielsweise der erfassten ausgetragenen Menge bestimmt werden.

Es ist auch möglich, dass neben der Erfassungseinrichtung eine Datenaustauscheinrichtung vorgesehen ist, wobei Erfassungseinrichtung und Datenaustauscheinrichtung in einer selben Baueinheit, insbesondere in ein und demselben Elektronik-Chip, integriert sein können. Die Datenaustauscheinrichtung ist beispielsweise dazu geeignet, in der Erfassungseinrichtung erfasste Größen an externe Datenverarbeitungseinrichtungen - insbesondere berührungsfrei - zu übertragen. Es kann dabei auch eine bi-direktionale Datenübertragung erfolgen. Hierbei kommt beispielsweise die Datenübertragung mittels Infrarot-Licht, über Transponder oder über Funkeinrichtungen in Frage. Mittels der Datenübertragung wird auch eine Ferndiagnose, Fernüberwachung und/oder Fernbehandlung innerhalb lokaler oder nicht-lokaler Funknetze ermöglicht. Soweit eine bi-direktionale Datenübertragung erfolgt, können auch von außen Abfragen an den Spender gerichtet und erfasste Daten abgefragt werden. Es ist auch möglich, Betriebszustand oder Betriebsparameter (Dauer von Betätigungssperren, Aufheben oder Aktivieren einer Betätigungssperre, Volumen eines Austraghubes, ...) von außen einzustellen, einzuspielen bzw. zu ändern, z. B. gemäß einer individuellen ärztlichen Verordnung. Die externen Datenverarbeitungseinrichtungen können bevorzugt aus einer Basisstation und einer Auswertungsstation bestehen. Die Basisstation kann beim Patienten stehen und neben Aufbewahrungs-, Stromversorgung, Speicher- und Anzeigefunktionen ein Datenübertragungs-Modul, z. B. ein GSM-Modem enthalten, über das eine Faxübertragung zu einem PC des Arztes oder einem Institut durchgeführt wird, das die Ergebnisse der Behandlung auswertet und ggf., davon abgeleitet, korrigiert.

Darüber hinaus kann am Betätigungsmittel eine Sensoreinrichtung vorgesehen sein, über die eine Authentifikation des Benutzers ermöglicht wird. Insbesondere die Authentifikation eines Benutzers über unveränderliche, körpereigene Merkmale, beispielsweise Fingerabdruck und Augenhintergrund, aber auch die Eingabe eines sogenannten PIN-Codes an einer geeigneten Eingabe-Schnittstelle ist geeignet, die Berechtigung zur Benutzung des Spenders für jede einzelne Betätigung zu überprüfen. Hiermit wird insbesondere für die Fälle, in denen der pharmazeutische Wirkstoff einer sehr genauen Beobachtung und strengen Verschreibungsregeln unterliegt, wie beispielsweise im Bereich der Opiate und anderer süchtig machender Arzneimittel, die Überprüfung des tatsächlichen Benutzers ermöglicht.

Besonders geeignet ist der Spender für die genaue Überwachung und Dokumentation sogenannter klinischer Tests, die bei jedem Medikament vor der Zulassung nötig sind. Er ermöglicht trotz höheren Herstellungsaufwandes erhebliche Einsparrungen bei der Überwachung der genauen Anwendung.

Die vorstehenden und weitere Merkmale gehen außer aus den Ansprüchen auch aus der Beschreibung und den Zeichnungen hervor, wobei die einzelnen Merkmale jeweils für sich allein oder zu mehreren in Form von Unterkombinationen bei einer Ausführungsform der Erfindung und auf anderen Gebieten verwirklicht sein und vorteilhafte sowie für sich schutzfähige Ausführungen darstellen können, für die hier Schutz beansprucht wird.

### Kurz-Beschreibung der Zeichnungen

Die Erfindung wird nachfolgend anhand eines in der einzigen Zeichnung dargestellten Ausführungsbeispiels näher erläutert.
- Fig. 1: zeigt einen erfindungsgemäßen Spender mit einer Erfassungseinrichtung und mehreren Sensoren in schematischer Darstellung,
- Fig. 2: zeigt eine perspektivische Ansicht eines Spenders und einer zugehörigen Basisstation,
- Fig. 3: ist ein mit Symbolen versehenes Blockschaltbild, anhand dessen das Zusammenwirken des Spenders mit einer Basisstation und einer Auswertungsstation erläutert wird.

### Beschreibung eines bevorzugten Ausführungsbeispiels

Die Zeichnung zeigt in schematisch teilgeschnittener Darstellung ein Ausführungsbeispiel eines erfindungsgemäßen Spenders, wie er beispielsweise für den Austrag von Medien geeignet ist, die in die Nase einer zu behandelnden Person appliziert werden und wenigstens einen pharmazeutischen Wirkstoff oder eine pharmazeutische Wirkstoffkombination enthalten. Das Medium 11 ist in einem Vorratsbehälter 21 des Spenders 20 bevorratet. Der Vorratsbehälter 21 ist im Inneren des Spenders 20 gehalten. Von dem Vorratsbehälter 21 führt über eine Fördereinrichtung 24, die den Austrag aus dem Vorratsbehälter erzeugt, ein Medienpfad 22 zu der Austragöffnung 23 des Spenders, die im Bereich einer Nasenolive 25 ausgebildet ist. Die Nasenolive 25 dient dem Ansatz des Spenders im Bereich der Nasenlöcher der zu behandelnden Person. Die Austragöffnung 23 ist für einen zerstäubten Austrag des Mediums als Zerstäuberdüse ausgebildet. Die Fördereinrichtung 24 fördert das Medium aus dem Vorratsbehälter 21 zur Austragöffnung 23 und ist als einfachwirkende Schubkolbenpumpe ausgebildet. Eine solche Pumpe kann in relativ einfacher Weise Medium genau dosieren. Sie hat einen in einem Pumpenzylinder 30 geführten Kolben 31 und saugt das Medium 11 über ein Saugröhrchen 33 an.

Die Betätigung des Spenders 20 erfolgt über ein als Betätigungsdrücker ausgebildetes Betätigungsmittel 26, das bei jeder Betätigung einen Austraghub erzeugt.

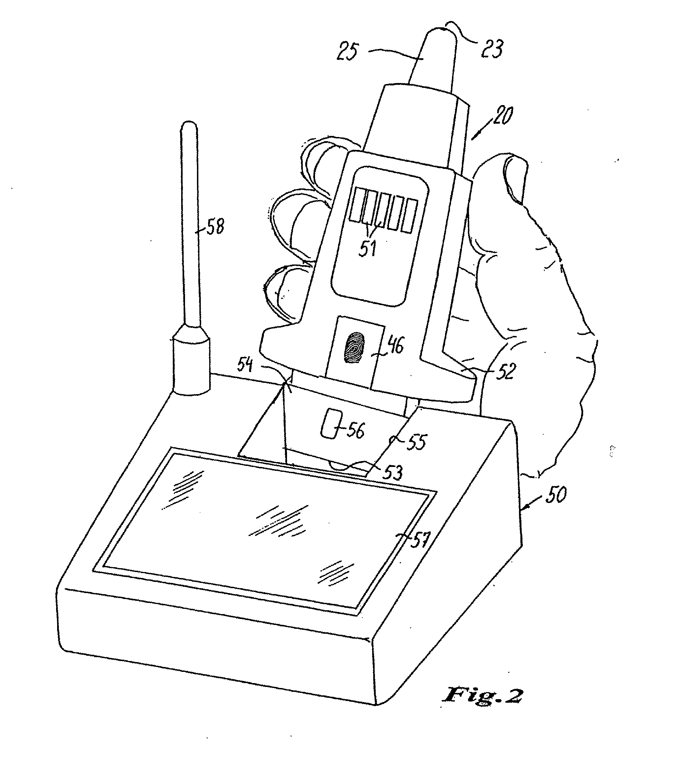

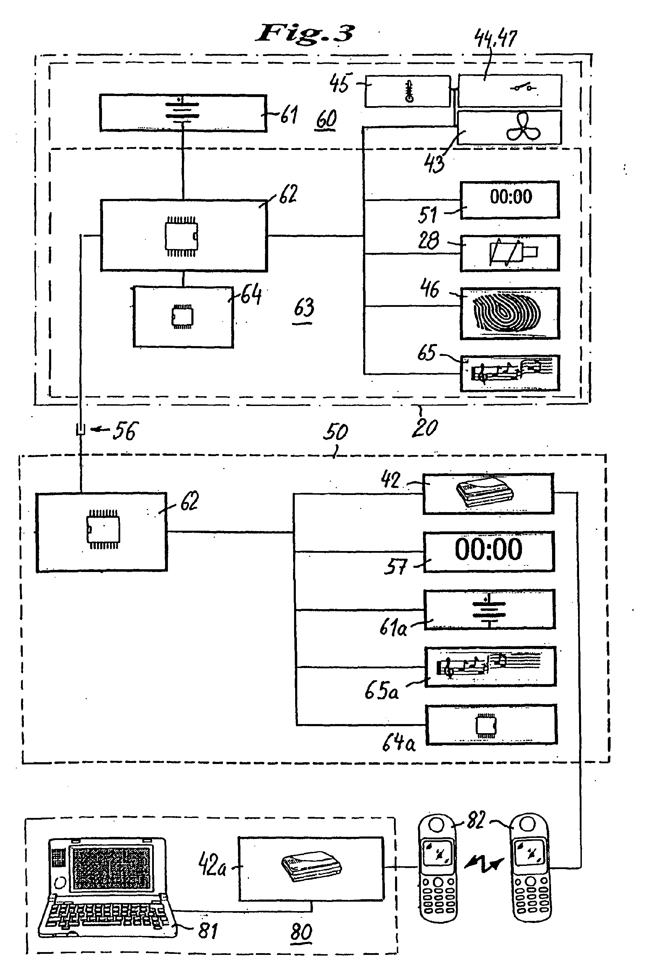

Eine Erfassungseinrichtung 40 umfasst eine Auswerteeinheit 41 und eine Datenübertragungseinrichtung 42. Die Datenübertragungseinrichtung 42 ist in der Lage, Daten der Auswerteeinheit 41 an eine und von einer externen Datenverarbeitungs-Basisstation 50 zu übermitteln (vgl. Fig. 2 und 3). Bei den Daten kann es sich sowohl um aufgezeichnete Daten handeln, als auch um programmierbare Vorgaben, die von der Basisstation 50 zur Auswerteeinheit 41 übertragen werden, um beispielsweise in Abhängigkeit von vorgegebenen individuell einstellbaren Dosierangaben den Austrag aus dem Spender zu beeinflussen.

Zur Erfassung der ausgetragenen Mediummenge umfasst die Erfassungseinrichtung 40 beim Ausführungsbeispiel einen Durchflussmengenmesser 43 und einen Wegsensor 47. Der Wegsensor 47 erfasst den Betätigungsweg des Betätigungsmittels 26 und damit den Hubweg der Kolbenpumpe 24, die daran gekoppelt ist. Dieser Hubweg dient als Maß für die ausgetragene Menge Medium. Der Durchflussmengenmesser 43, der beispielsweise als Differenzdruckmesser oder aber als induktiver oder thermischer Durchflussmengenmesser ausgebildet sein kann, liefert im Gegensatz zum indirekten Maß für die Austragsmenge, die über den Wegsensor 47 gemessen wird, ein direktes Maß für die tatsächlich ausgetragene Medienmenge. Der Durchflussmengenmesser 43 ist daher insbesondere in einem Bereich des Medienpfades 22 angeordnet, der möglichst nahe der Austragöffnung 23 ist und bei dem es möglichst wenig Möglichkeiten für Leckagen und andere Störungen gibt.

Ebenfalls in unmittelbarer Nähe zur Austragöffnung 23 im Bereich der Nasenolive ist ein Temperatursensor 45 als Messfühler angeordnet. Der Temperatursensor 45 ist so angeordnet, dass zwischen ihm und der äußeren Umgebung des Spenders 20 eine möglichst geringe thermische Kapazität ausgebildet ist, damit er möglichst rasch Temperaturänderungen der Umgebung erfassen kann. Dadurch wird es möglich, festzustellen, ob die Nasenolive im Bereich der Nase des Patienten angeordnet ist, da hier aufgrund des Luftstromes der ausgeatmeten Luft die Temperatur ziemlich genau auf einen Wert im Bereich der Körpertemperatur (ca. 36°C bis 42°C) ansteigen müsste. Darüber hinaus kann mittels des Temperatursensors 45 auch erfasst werden, ob die Transport- und Lagertemperatur sich in Bereichen bewegt, die dem Medium angepasst sind. Dazu können die Temperaturdaten in einer vorgegebenen zeitlichen Abhängigkeit von der Temperaturempfindlichkeit des Medikaments gespeichert werden. Diese aufgezeichneten Daten können vor Abgabe des Spenders mit dem darin enthaltenen Medium an den Kunden über die Datenübertragungseinrichtung 42 ausgelesen und überprüft werden. So kann insbesondere bei Medikamenten, bei denen die Haltbarkeit sehr begrenzt ist und die sehr temperaturabhängig sind, kontrolliert werden, ob die Qualität des Mediums nicht beeinträchtigt ist. Die Datenübertragung von der Datenübertragungseinrichtung zur Feststation kann dabei über entsprechende Infrarotschnittstellen oder Transponder, oder aber auch galvanisch durch Kontakte oder durch Herstellung einer Kabelverbindung erfolgen.

Darüber hinaus kann der Auswerteeinheit 41 auch noch das Signal eines Endlagenschalters 44 zugeführt werden, der erfasst, ob ein kompletter Austragshub der Fördereinrichtung 24 durchgeführt wurde. Diese Möglichkeit kann dazu genutzt werden, um in der Auswerteeinheit 41 der Erfassungseinrichtung 40 einen Vergleich mit den ausgewerteten Signalen des Durchflussmengenmessers 43 bzw. des Wegsensors 47 durchzuführen. Über einen Abgleich dieser Sensorsignale kann beispielsweise eine Eichung derselben vorgenommen werden. Es ist nämlich über die Dosiergenauigkeit der Kolbenpumpe möglich, darauf zu schließen, ob das Volumen der vom Durchflussmengenmesser gemessenen Menge Medium dem Kolbenhubvolumen entspricht, der bei einem vollständigen Austraghub in dem entsprechenden Zylinder verdrängt wird. Hierzu sollte sich der Messunterschied in einer engen Grenze bewegen, da ansonsten von einem Defekt auszugehen ist, der gesonderter Prüfung bedarf. Eine entsprechende, beispielsweise optische und/oder akustische Fehlermeldung kann generiert werden. Darüber hinaus kann in der Auswerteeinheit 41 auch erfasst werden, wer den Spender 20 benutzt. Dies kann beispielsweise über einen den Fingerabdruck des Benutzers an dem Betätigungsmittel 26 erfassenden Fingerabdruck-Sensor 46 erfolgen. Auch andere Sensoren, beispielsweise ein Sensor, der im Bereich der Austragsöffnung angeordnet ist und z. B. geeignet ist, den Augenhintergrund zu erfassen, sind denkbar. Der letztgenannte Sensor kann insbesondere dann verwendet werden, wenn die Applikation im Bereich des Auges eines Patienten erfolgen soll. Es ist so möglich, nur bestimmten Personen die Benutzung des Spenders zu ermöglichen.

In der Auswerteeinheit 7 kann, besonders wenn außer der ausgetragenen Medienmenge auch noch die Betätigungszeit erfasst wird, mengen- und zeitabhängig auch die Betätigung des Spenders begrenzt oder gesperrt werden. Hierzu dient im dargestellten Ausführungsbeispiel ein Servo 28. Dieser kann die Dosierung, also der maximal mögliche Hubweg der Fördereinrichtung 24, der durch den Betätigungsweg des Betätigungsmittels bestimmt ist, begrenzen. Dabei kann die Begrenzung so erfolgen, dass in einem Extremzustand der Begrenzung die Wirkung einer Betätigungssperre erreicht wird, so dass überhaupt ein Medienaustrag verhindert wird und im anderen Extrem ein vollständiger Kolbenhub durchführbar ist, um den Austrag eines oder mehrerer Kolbenhübe zu ermöglichen. Die Betätigungssperre, die z. B. wie in der EP 1125637 A (entsprechend US Ser. No. 09/780287), auf die Bezug genommen wird, ausgebildet sein kann, soll verhindern, dass eine Überdosierung des Medikamentes beim Patienten erzeugt wird. Andererseits kann auch beispielsweise durch gleitende Mittelwertbildung die austragbare Menge Medium so bestimmt werden, das sie eine bestimmte maximale Konzentration nicht übersteigt. Andererseits kann es dabei zugelassen werden, dass immer auch geringfügige Mengen Medium nachverabreicht werden können, die so bestimmt sind, dass die maximale Konzentration - in Abhängigkeit beispielsweise einer angenommenen Abbaurate im Körper - nicht überschritten wird. Dies ermöglicht es, bei einem Patienten auch über eine längere Zeit hinweg nur geringe Konzentrationsschwankungen durch häufige Verabreichungen des Medikamentes zu erzielen und zugleich eine Überdosierung zu verhindern.

Neben der gleitenden Mittelwertbildung kommen natürlich auch andere Verfahren in Betracht, die maximale Austragsmenge, die beim nächsten Austragshub ausgetragen werden kann, zu bestimmen. So kann es z. B. in Frage kommen, zunächst über einen bestimmten Zeitraum einen weiteren Austrag zu sperren und dann über einen bestimmten weiteren Zeitraum hinweg die maximal austragbare Dosis kontinuierlich zu erhöhen bis einer oder mehrere komplette Betätigungshübe ausgetragen werden können.

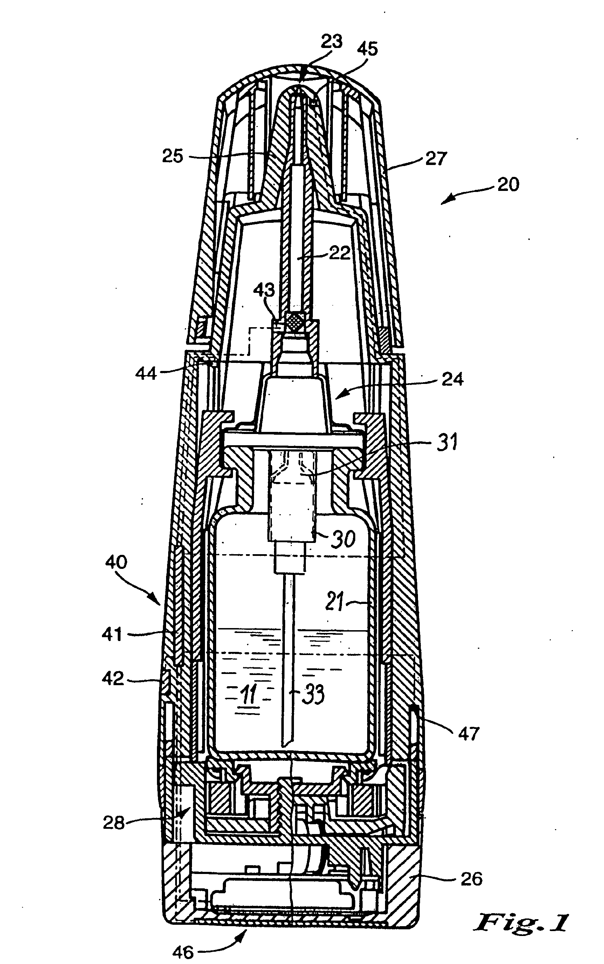

Figur 2 zeigt den Spender 20, der per Hand aus einer Basisstation 50, die zu seiner Aufbewahrung dient, zu seiner Benutzung herausgenommen werden kann. Am Spender sind Anzeigen 51 in Form von LCD-Feldern vorgesehen, über die die Daten angezeigt werden können. Der Spender 20 hat ferner seitlich vorstehende Schultern 52, die zwischen zwei Fingern gehalten werden können, wenn der Spender durch Druck auf die Betätigungsfläche 53 manuell betätigt wird. Auf der Betätigungsfläche 53 kann sich auch der Sensor 46 der Identifikationseinrichtung durch Erkennung des Fingerabdrucks des Benutzers befinden (siehe Figur 1) oder, wie in Figur 2 gezeigt, auf einem gesonderten Feld an einer Seite des Spenders.

In dem Betätigungsabschnitt 54 des Spenders, der in einer entsprechenden Ausnehmung 55 der Basisstation 50 aufgenommen werden kann, ist eine Anschlussfläche 56 vorgesehen, über die Daten von dem Spender zur Basisstation übertragen werden können. Dies kann beispielsweise durch Optokopplung, galvanischen Anschluss, induktiv oder per Transponder geschehen.

Die Basisstation enthält ein Anzeigefeld 57, beispielsweise einen LCD-Schirm, auf dem größere Datenmengen angezeigt werden können. Von außen ist ferner eine Antenne 58 für die Datenfernübertragung zu erkennen, die anschließend anhand von Figur 3 erläutert wird.

Figur 3 zeigt das Datenerfassungssystem nach der Erfindung in schematischer Blockdarstellung. Im Spender 20 ist, ggf. in einem für jeden Benutzer oder jede Mediencharge austauschbaren "Wegwerfteil" 60, eine Energieversorgung 61 in Form einer Batterie oder eines Akku, der über die Basisstation aufgeladen werden kann, vorhanden. Ferner sind darin diejenigen Sensoren enthalten, die mit dem Medikament und ggf. dem Benutzer in Berührung kommen und daher auszutauschen sind, und zwar der Temperatursensor 45, Wegsensor bzw. Endlagenschalter 44, 47 und Durchflusssensor 43. Sie liefern ihre Messsignale an einen Mikrokontroller 62, der sich in dem stets wiederverwendeten Teil 63 des Spenders befindet. Der Mikrokontroller oder Mikrocomputer in Form eines Chips ist mit einem elektronischen Speicher 64 verbunden. Er steuert die Abfrage der Daten von den Sensoren und die übrigen Erfassungs- und Spenderfunktionen einschließlich Anzeige, Identifikation, Servofunktionen und Datenweiterleitung. Dazu sind in Figur 3 gezeigt: eine Anzeige 51 (siehe auch Figur 2), der Identifikationssensor 46, ein akustischer Signalgeber, z. B. ein Summer 65, und eine Servoeinrichtung 28, über die, wie schon anhand von Figur 1 beschrieben, Betätigungssperren, Hublängenveränderungen etc. vorgenommen werden können.

Der Datenausgang aus dem Spender wird über die bereits erwähnte Anschlussfläche 56 vorgenommen, die die Daten in die Basisstation 50 eingibt. Dort ist ebenfalls ein Mikrokontroller oder Mikrocomputer 62 zusammen mit der nicht dargestellten zugehörigen Stromversorgung etc. vorgesehen, der folgende der darin enthaltenen Teile bzw. Einheiten beschickt und koordiniert: eine Datenfernübertragungseinrichtung 42, beispielsweise ein GSM-Modem, dass heißt einen Datenanschluss an das Mobilfunknetz, die Anzeige 57, eine Stromversorgung 61 a (Batterie oder netzbetrieben), eine akustische Anzeige 65a und einen Speicher 64a.

Die Basisstation kann die Daten von einem oder mehreren Spendern auch in größerem Umfang speichern und verarbeiten sowie an die einzelnen Mikrocomputer der Spender Befehle weiterleiten, die entweder generelle Programmbefehle sind oder einzelne in Auswirkung der von den Sensoren eingehenden Daten erzeugte Ausführungsbefehle, z. B. für die Servoeinrichtung 28.

Gleichzeitig dient die Basisstation dazu, über die Datenfernübertragungseinrichtung 42 eine externe Station 80 mit den aus dem Spender gewonnen und dort bzw. in der Basisstation verarbeiteten Daten zu versorgen oder Daten von der externen Station 80 in die Basisstation oder den Spender einzulesen. Dies erfolgt beim Ausführungsbeispiel mittels zweier Mobiltelefone 82 mit Datenübertragungs-Funktion und ein weiteres GSM-Modem 42a in der externen Station 80, an die beispielsweise ein PC (Personalcomputer) 81 angeschlossen sein kann. Dieser kann selbständig oder bedient von einem Arzt oder einer entsprechenden Laborkraft die gewonnenen Daten auswerten bzw. den Spender entsprechend automatisch ansteuern und programmieren, um ein ideales Behandlungsergebnis zu erzielen.

## Patentansprüche

1. Spender für Medien, vorzugsweise wenigstens einen pharmazeutischen Wirkstoff enthaltende Fluide, mit einem Vorratsbehälter und einer Austragsöffnung, wobei ein Medienpfad den Vorratsbehälter mit der Austragsöffnung verbindet und wobei eine Fördereinrichtung zum Fördern von Medium aus dem Vorratsbehälter zur Austragsöffnung vorgesehen ist, mittels der in Abhängigkeit von der Betätigung eines Betätigungsmittels ein Medienaustrag erfolgt, und mit einer Erfassungseinrichtung (40) mit wenigstens einem Sensor (43-47) für die Erfassung der ausgetragenen Menge des Mediums, **dadurch gekennzeichnet, dass** die Fördereinrichtung (24) eine Kolbenpumpe (32) ist, dass der Sensor (43) ein Durchflussmengenmesser für die Messung der Menge des ausgetragenen Mediums ist, der im Medienpfad (22) zwischen der Fördereinrichtung (24) und der Austragsöffnung (23) angeordnet ist und dass die Erfassungseinrichtung (40) einen Speicher für die bezüglich einer Anzahl von Betätigungen des Spenders (20) erfassten Informationen sowie eine Anzeige (51) zur Datenanzeige aufweist.

2. Spender nach Anspruch 1, **dadurch gekennzeichnet, dass** der Durchflussmengenmesser (43) ein induktiver, thermischer oder nach dem Druckdifferenzmessverfahren arbeitender Durchflussmengenmesser ist.

3. Spender nach einem der Ansprüche 1 oder 2, **dadurch gekennzeichnet, dass** der Durchflussmengenmesser einen Messbereich von 30 bis 150 µl Medienvolumen, insbesondere innerhalb von 50 bis 100 Millisekunden Strömungsdauer, aufweist.

4. Spender nach einem der vorhergehenden Ansprüche, **dadurch gekennzeichnet, dass** ein Endlagenschalter (44) vorgesehen ist, der das Erreichen eines oberen Totpunktes der Pumpe (32), der einem vollständigen Pumpenhub entspricht, erfasst und ggf. Mittel vorgesehen sind, die zusätzlich das Verlassen einer betätigungsfrei eingenommenen, unteren Totpunktlage der Pumpe erfassen.

5. Spender nach einem der vorhergehenden Ansprüche, **dadurch gekennzeichnet, dass** ein Wegsensor (47) vorgesehen ist, der den von der Pumpe zurückgelegten Austragshubweg erfasst, wobei die Erfassungseinrichtung bei Nichterreichen eines oberen Totpunktes einen Teilaustrag von Medium registriert und ggf. entsprechende Signale erzeugt.

6. Spender nach einem der vorhergehenden Ansprüche, **dadurch gekennzeichnet, dass** die elektronische Erfassungseinrichtung zur Ermittlung und ggf. Speicherung eines Zeitwertes jeder Betätigung des Spenders (20) ausgebildet ist.

7. Spender nach einem der vorhergehenden Ansprüche, **dadurch gekennzeichnet, dass** in Abhängigkeit von den von der Erfassungseinrichtung (40) erfassten Größen das maximale Austragsvolumen einer Betätigung des Spenders (20) bestimmbar und ggf. steuerbar ist.

8. Spender nach einem der vorhergehenden Ansprüche, **dadurch gekennzeichnet, dass** im Bereich der Austragöffnung (23) ein Messfühler (45) angeordnet ist, der insbesondere eine thermische, optische oder Feuchte-Messung durchführt, der bevorzugt an der Spitze einer Nasenolive, dessen Messsignal der Erfassungseinrichtung zur Steuerung dient.

9. Spender nach einem der vorhergehenden Ansprüche, **dadurch gekennzeichnet, dass** eine in Abhängigkeit von in der Erfassungseinrichtung (40) erfassten Größen schaltbare, den Austrag von Medium sperrende oder freigebende Betätigungssperre vorgesehen ist, die ggf. eine erneute Betätigung des Spenders (20) in Abhängigkeit wenigstens des Austragsvolumens vorhergehender Austraghübe und der seither verstrichenen Zeit verhindert.

10. Spender nach einem der vorhergehenden Ansprüche, **dadurch gekennzeichnet, dass** der Spender eine Datenaustauscheinrichtung aufweist, durch die wenigstens in der Erfassungseinrichtung (40) erfasste Größen an eine Spender-externe Datenverarbeitungseinrichtung übertragbar sind, die vorzugsweise eine Basisstation (50) enthält, mit der der Spender mittels Kurz-Entfernungsübertragung oder galvanisch in Datenaustausch steht und die ggf. mit einer Auswertestation per Fern-Datenübertragung (42, 80, 42a) kommuniziert und vorzugsweise für eine bi-direktionale Kommunikation ausgebildet ist.

11. Verfahren zur Datenerfassung bei einem Spender (20) nach einem der vorhergehenden Ansprüche, **dadurch gekennzeichnet, dass** durch den Sensor (43 bis 47) wenigstens eines der folgenden Daten in Form elektrischer Signale erfasst wird: Austragsmenge, Hubweg der Pumpe, Betätigungszeit bzw. -dauer, Temperatur, Helligkeit, Feuchtigkeit, und dass diese Daten verarbeitet und gespeichert sowie ggf. an externe Datenverarbeitungseinrichtungen (50, 80) übermittelt werden und dass ggf. in Abhängigkeit von der Datenerfassung die Funktion des Spenders (10) gesteuert bzw. beeinflusst wird.

12. Verfahren nach Anspruch 11, **dadurch gekennzeichnet, dass** in Abhängigkeit von Messsignalen des Sensors (43 bis 47) wenigstens eine der nachfolgenden Maßnahmen ergriffen wird:
- es wird auf eine Applikation des Mediums an einem vorgesehenen Applikationsort geschlossen;
- es wird eine Betätigungssperre aktiviert oder deaktiviert;
- die Betätigung des Spenders (20) wird von der Erfassungseinrichtung (40) als Medienaustrag gewertet.

13. Datenerfassungssystem mit einem Spender (20) nach einem der Ansprüche 1 bis 10, **gekennzeichnet durch**
- eine Speichereinrichtung (64, 64a) für die Daten des Sensors (43, 44, 47), die Eigenschaften der ausgetragenen bzw. einem Benutzer applizierte Menge repräsentieren,
- eine Datenverarbeitungseinrichtung (62) für die Daten,
- sowie wenigstens eines der folgenden Merkmale:
- eine Datenübertragungseinrichtung (42) für die ggf. überarbeiteten Daten an und/oder von einer Basisstation (50),
- eine Anzeigeeinrichtung (51, 57) zur Anzeige der ggf. verarbeiteten Daten,
- eine Identifizierungseinrichtung (46) für einen autorisierten Benutzer,
- wenigstens einen Sensor zur Erkennung des Applikationsortes (45),
- wenigstens einen Sensor zur Erkennung des Applikationserfolges,
- eine Sensoreinrichtung (28) zur Beeinflussung wenigstens einer Funktion des Spenders (20),
- eine Fern-Datenübertragungseinrichtung (42, 42a, 58, 82) zur Übertragung von Daten zwischen der Basisstation (50) und einer weiteren externen Station (80).

## Claims

1. Dispenser for media, preferably fluids containing at least one active pharmaceutical agent, having a supply container and a discharge opening, where a media path connects the supply container to the discharge opening and where a conveying device is provided for conveying of medium out of the supply container to the discharge opening, by means of which media are discharged depending on the actuation of an actuating means, and having a recording device (40) with at least one sensor (43-47) for recording of the discharged quantity of the medium, **characterized in that** the conveying device (24) is a piston pump (32), **in that** the sensor (32) is a flow meter for measuring the quantity of the discharged medium and which is arranged in the media path (22) between the conveying device (24) and the discharge opening (23), and **in that** the recording device (40) has a memory for the information recorded with regard to the number of actuations of the dispenser (20) and a display (51) for displaying the data.

2. Dispenser according to Claim 1, **characterized in that** the flow meter (43) operates inductively, thermally or in accordance with the pressure differential measurement method.

3. Dispenser according to one of Claims 1 or 2, **characterized in that** the flow meter has a measuring range from 30 to 150 µl of medium volume, in particular within 50 to 100 milliseconds of flow duration.

4. Dispenser according to one of the preceding claims, **characterized in that** a limit switch (44) is provided that records the reaching of a top dead center of the pump (32), corresponding to a complete pump stroke, and where means are provided if necessary that additionally record the leaving of a bottom dead center position of the pump assumed without actuation.

5. Dispenser according to one of the preceding claims, **characterized in that** a travel sensor (47) is provided that records the discharge stroke travel covered by the pump, where the recording device registers a partial discharge of medium when a top dead center has not been reached and if necessary generates appropriate signals.

6. Dispenser according to one of the preceding claims, **characterized in that** the electronic recording device is designed for ascertaining and if necessary storing a time value for each actuation of the dispenser (20).

7. Dispenser according to one of the preceding claims, **characterized in that** depending on the quantities recorded by the recording device (40) the maximum discharge volume of an actuation of the dispenser (20) can be determined and if necessary controlled.

8. Dispenser according to one of the preceding claims, **characterized in that** a measuring sensor (45) is arranged in the area of the discharge opening (23) and in particular performs a thermal, optical or moisture measurement and is preferably arranged on the tip of a nose olive, the measuring signal of which is used by the recording device for control.

9. Dispenser according to one of the preceding claims, **characterized in that** an actuation lock is provided for blocking or enabling the discharge of medium, is switchable depending on the quantities recorded by the recording device (40) , and if necessary prevents any further actuation of the dispenser (20) depending on at least the discharge volume of previous discharge strokes and on the time passed since then.

10. Dispenser according to one of the preceding claims, **characterized in that** the dispenser has a data exchange device by which at least the quantities recorded in the recording device (40) are transferable to a data processing device external to the dispenser and preferably containing a base station (50) with which the dispenser is in data-exchanging contact by means of short-distance transmission or galvanically and if necessary communicates with an evaluation station by remote data transmission (42, 80, 42a) and is designed preferably for bidirectional communication.

11. Method for data recording in a dispenser (20) according to one of the preceding claims, **characterized in that** at least one of the following data items is recorded by the sensor (43 to 47) in the form of electrical signals: discharge quantity, stroke travel of pump, actuation time and duration, temperature, brightness, moisture, and **in that** this data is processed and stored and if necessary transferred to external data processing devices (50, 80), and **in that** the function of the dispenser (10) is controlled or influenced depending on the data recording.

12. Method according to Claim 11, **characterized in that** depending on measurement signals of the sensor (43 to 47) at least one of the following measures is taken:
- an application of the medium at an intended application location is concluded;
- an actuation lock is activated or deactivated;
- the actuation of the dispenser (20) is evaluated by the recording device (40) as a discharge of medium.

13. Data recording system with a dispenser (20) according to one of Claims 1 to 10, **characterized by**
- a memory device (64, 64a) for the data of the sensor (43, 44, 47) representing properties of the quantity discharged or applied to a user,
- a data pocessing device (62) for the data,
- and at least one of the following features:
- a data transmission device (42) for any data revised to and/or from a base station (50),
- a display device (51, 57) for display of any data processed,
- an identification device (46) for an authorized user,
- at least one sensor for detection of the application location (45),
- at least one sensor for detection of the application success,
- a sensor device (28) for influencing at least one function of the dispenser (20),
- a remote data transmission device (42, 42a, 58, 82) for transfer of data between the base station (50) and a further external station (80).

## Revendications

1. Distributeur pour fluides, de préférence pour un fluide contenant un agent actif pharmaceutique, avec un réservoir et un orifice de distribution, sachant qu'un conduit de fluide relie le réservoir à l'orifice de distribution et qu'est prévu un dispositif de refoulement pour refouler le fluide hors du réservoir vers l'orifice de distribution et au moyen duquel a lieu une distribution de fluide en fonction de l'actionnement d'un moyen d'actionnement, et avec un dispositif enregistreur (40) avec au moins un capteur (43 à 47) pour enregistrer la quantité de fluide distribuée, **caractérisé en ce que** le dispositif de refoulement (24) est une pompe à piston (32), que le capteur (43) est un débitmètre pour mesurer la quantité de fluide distribuée, qui est placé dans le conduit de fluide (22) entre le dispositif de refoulement (24) et l'orifice de distribution (23), et que le dispositif enregistreur (40) présente une mémoire pour les informations recueillies concernant un nombre d'actionnements du distributeur (20) ainsi qu'un écran (51) pour afficher les données.

2. Distributeur selon la revendication 1, **caractérisé en ce que** le débitmètre (43) est un débitmètre inductif, thermique ou travaillant selon la méthode de mesure de pressions différentielles.

3. Distributeur selon l'une des revendications 1 ou 2, **caractérisé en ce que** le débitmètre présente une plage de mesure de 30 à 150 µl de volume de fluide, en particulier dans un intervalle de 50 à 100 millisecondes de durée d'écoulement.

4. Distributeur selon l'une des revendications précédentes, **caractérisé en ce que** sont prévus un interrupteur de bout de course (44) qui enregistre l'atteinte d'un point mort haut de la pompe (32) correspondant à une course de pompe complète, et le cas échéant des moyens qui enregistrent en outre l'abandon d'une position au point mort basse de la pompe, prise sans actionnement.

5. Distributeur selon l'une des revendications précédentes, **caractérisé en ce qu'**est prévu un capteur de course (47) qui enregistre la course de distribution parcourue par la pompe, sachant qu'en cas de non-atteinte d'un point mort haut, le dispositif enregistreur enregistre une distribution partielle de fluide et génère le cas échéant des signaux correspondants.

6. Distributeur selon l'une des revendications précédentes, **caractérisé en ce que** le dispositif enregistreur électronique est conçu pour détecter et le cas échéant enregistrer une valeur-temps de chaque actionnement du distributeur (20).

7. Distributeur selon l'une des revendications précédentes, **caractérisé en ce que** le volume maximal de distribution d'un actionnement du distributeur (20) peut être déterminé et éventuellement contrôlé en fonction des grandeurs enregistrées par le dispositif enregistreur (40).

8. Distributeur selon l'une des revendications précédentes, **caractérisé en ce que** dans la zone de l'orifice de distribution (23) est placé un capteur (45), de préférence à la pointe d'une olive nasale, qui effectue en particulier une mesure thermique, optique ou hygrométrique, dont le signal de mesure sert au dispositif enregistreur pour la commande.

9. Distributeur selon l'une des revendications précédentes, **caractérisé en ce qu'**est prévu un blocage d'actionnement commutable qui bloque ou débloque la distribution de fluide en fonction des grandeurs enregistrées dans le dispositif enregistreur (40), et qui le cas échéant empêche un nouvel actionnement du distributeur (20) en fonction d'au moins le volume distribué lors des courses de distribution précédentes et du temps écoulé depuis.

10. Distributeur selon l'une des revendications précédentes, **caractérisé en ce que** le distributeur présente un dispositif d'échange de données, au moyen duquel les grandeurs saisies au moins dans le dispositif enregistreur (40) sont transmissibles à un dispositif externe de traitement des données du distributeur, et qui comprend de préférence une station de base (50), avec laquelle le distributeur se trouve en échange de données au moyen d'une transmission à courte distance ou galvaniquement, et qui le cas échéant communique avec une station d'exploitation par télétransmission de données (42, 80, 42a) et est de préférence conçue pour une communication bidirectionnelle.

11. Procédé de saisie de données sur un distributeur (20) selon l'une des revendications précédentes, **caractérisé en ce que** par le capteur (43 à 47) au moins l'une des données suivantes est saisie sous forme de signaux électriques : quantité distribuée, course de la pompe, moment ou durée d'actionnement, température, luminosité, humidité, et que ces données sont traitées, enregistrées et transmises le cas échéant à un dispositif externe de traitement de données (50, 80), et que la fonction du distributeur (10) est le cas échéant commandée ou influencée en fonction de la saisie des données.

12. Procédé selon la revendication 11, **caractérisé en ce qu'**en fonction des signaux de mesure du capteur (43 à 47) au moins l'une des actions suivantes est appliquée :
- il est conclu qu'une application du fluide a été effectuée à un lieu d'application prévu ;
- un blocage d'actionnement est activé ou désactivé ;
- l'actionnement du distributeur (20) est considéré comme distribution de fluide par le dispositif enregistreur (40).

13. Système de saisie de données avec un distributeur (20) selon l'une des revendications 1 à 10, **caractérisé par**
- un dispositif de mémorisation (64, 64a) pour les données du capteur (43, 44, 47) qui représentent les caractéristiques de la quantité distribuée ou appliquée par un utilisateur
- un dispositif de traitement de données (62) pour les données,
- ainsi que par au moins l'une des particularités suivantes :
- un dispositif de transmission de données (42) pour transmettre les données éventuellement modifiées à et/ou depuis une station de base (50),
- un dispositif d'affichage (51, 57) pour afficher les données éventuellement traitées,
- un dispositif d'identification (46) pour un utilisateur autorisé,
- au moins un capteur pour reconnaître le lieu d'application (45),
- au moins un capteur pour reconnaître le succès de l'application,
- un dispositif détecteur (28) pour influencer au moins une fonction du distributeur (20),
- un dispositif de télétransmission de donnée (42, 42a, 58, 82) pour la transmission de données entre la station de base (50) et une autre station externe (80).
